# EUROPÄISCHE PATENTSCHRIFT

(11) **EP 1 910 230 B1**
(45) Veröffentlichungstag und Bekanntmachung des Hinweises auf die Patenterteilung: **09.09.2009**
(21) Anmeldenummer: 06761762.1
(22) Anmeldetag: 05.07.2006
(51) Int. Cl.: C02F 1/48, C02F 11/04, C12M 1/42

(54) **VORRICHTUNG UND VERFAHREN ZUR BEHANDLUNG VON BIOMASSE MIT PERMANENTMAGNETEN**
DEVICE AND METHOD FOR THE TREATMENT OF BIOMASSES WITH PERMANENT MAGNETS
DISPOSITIF ET PROCEDE POUR TRAITER UNE BIOMASSE A L'AIDE D'AIMANTS PERMANENTS

(30) Priorität: 07.07.2005 DE 102005031873
(43) Veröffentlichungstag der Anmeldung: 16.04.2008
(73) Patentinhaber: Meier, Tonio, 86459 Gessertshausen (DE)
(72) Erfinder: Meier, Tonio, 86459 Gessertshausen (DE)
(74) Vertreter: Schlotter, Alexander Carolus Paul
(86) Internationale Anmeldenummer: PCT/DE2006/001160
(87) Internationale Veröffentlichungsnummer: WO 2007/006269

(56) Entgegenhaltungen:
- EP-A- 0 902 444
- EP-A1- 0 616 977
- FR-A- 2 681 062
- GB-A- 2 298 378
- JP-A- 2001 113 200
- JP-A- 2003 326 274
- US-A- 4 134 829
- US-A- 5 161 512

## Beschreibung

Die vorliegende Erfindung betrifft eine Vorrichtung zur Behandlung von Biomasse, insbesondere Klärschlamm nach dem Oberbegriff des Anspruchs 1 sowie ein Verfahren zur Behandlung von Biomasse, insbesondere Klärschlamm nach dem Oberbegriff des Anspruchs 6.

### Stand der Technik

Die vorliegende Erfindung wird nachfolgend am Beispiel der Behandlung von Klärschlamm näher erläutert. Gleichwohl bezieht sich die Erfindung allgemein auch auf die Behandlung beliebiger anderer Biomassen.

Gegenwärtig wird Abwasser meist dadurch geklärt, dass es mittels Rechen- und/oder Siebanlage zunächst grob mechanisch vorgereinigt wird. Das anfallende Rechengut kann ausgefault und kompostiert werden.

Das Abwasser gelangt dann in ein Absetzbecken, in dem Feststoffteilchen zu Boden sinken und dort als Suspension bzw. Schlamm gesammelt, ausgeräumt und einer Schlammfaulanlage zugeführt werden, in der unter Luftabschluss eine anaerobe bakterielle Zersetzung eintritt. Der fäulnisfähige Frischschlamm wird zersetzt, wobei ein Gas entsteht, welches beispielsweise in ein städtisches Gasversorgungsnetz eingeleitet werden kann. Der ausgefaulte Restschlamm kann als Dünger verwendet werden.

Die nach dieser mechanischen Reinigung im Abwasser noch enthaltenen Schmutz- und Fremdstoffe werden in einer anschliessenden biologischen Abwasserreinigung entfernt und aufgearbeitet Diese erfolgt aerob unter ausreichender Luftzufuhr: in einem oder mehreren Belebtschlammbecken schweben Bakterien im Abwasser und bauen organische Verunreinigungen ab. Der entstehende Belebtschlamm kann je nach spezifischem Gewicht an der Wasserobertläche oder dem Beckenboden der Belebungsbecken bzw.

Nachklärbecken gesammelt und ausgeräumt bzw. abgepumpt werden. Auch dieser Überschussschlamm wird in einer Schlammfaulanlage ausgefault, wobei wiederum Gas und ausgefaulter Restschlamm anfallen.

Damit fällt in einer Kläranlage in wenigstens einer, meist in mehreren Stufen; fäulnisfähiger Schlamm (Rechengut, Frischschlamm, Überschussschlamm, Belebtschlamm, Schwimmschlamm u.ä.) an, der nachfolgen insgesamt als Klärschlamm bezeichnet wird. Dieser kann ausgefault werden, wobei nutzbares Heizgas und ausgefaulter Restschlamm entstehen.

Dieser Restschlamm kann zwar einerseits als Dünger verwendet werden, muss jedoch, sofern er beispielsweise toxische Stoffe enthält, aufwendig in Deponien entsorgt oder verbrannt werden.

Es ist daher wünschenswert, einerseits den Heizwert und damit den Nutzen des Heizgases zu erhöhen und andererseits die Menge des anfallenden Restschlammes zu reduzieren. Auch bei der Gewinnung von Biogas aus anderen Biomassen ist eine Erhöhung des Heizwerts und eine Reduzierung der Restmasse vorteilhaft.

Hierzu ist es beispielsweise aus der DE 198 13 451 A1 bekannt, bei der biologischen Reinigung Ultraschall einzusetzen, der zum Aufschluss des Klärschlamms führt.

Alternativ schlagen die DE 33 14 863 A1, DE 199 55 219 A1 und DD 124913 vor, den Klärschlamm im Faulbehälter einem elektromagnetischen Wechselfeld auszusetzen. Dieses bewirkt eine zusätzliche Erwärmung des Schlamm, einen Zellaufschluss und eine Belebung von Mikrolebewesen, und erhöht so die Gasausbeute bei reduzierter Verweilzeit im Faulbehälter und Menge an Restschlamm.

Die DE 101 18 839 A1 schlägt gewissermassen eine Kombination beider Verfahren vor, indem Piezo-Füllkörper durch Ultraschall zur Erzeugung hochfrequenter elektromagnetischer Wechselfelder angeregt werden. Zusätzlich kann eine permanentmagnetische Schüttung vorgesehen sein.

Nachteilig verbrauchen die zur Erzeugung des elektromagnetischen Wechselfeldes erforderlichen Elektromagnete jedoch elektrische Energie. Zudem sind sie nur aufwendig in bestehende Anlagen zu integrieren und erfordern darüber hinaus eine beständige Regelung der erzeugten Feldstärken und eine Überwachung der Funktionalität. Diese Überwachung, Regelung und Energieversorgung ist angesichts der hohen Dauerbetriebszeiten von Kläranlagen und den dort herrschenden rauhen, insbesondere korrosiven Umgebungsbedingungen aufwendig, umständlich, teuer und störanfällig sowie schwer nachrüstbar. Gleiches gilt analog für den Einsatz von Ultraschall. Beide Verfahren weisen darüber hinaus unerwünschte Nebenwirkungen auf - so führt der Dauerbeschlag mit Ultraschall zur Ermüdung der Wasserrohre der Kläranlagen, die elektromagnetischen Wechselfelder können als Elektrosmog das Bedienungspersonal beeinträchtigen.

Das Permanentmagnetfeld der DE 10.1 18 839 A1 vermeidet diese Nachteile. Es handelt sich jedoch um ein homogenes Magnetfeld, dessen Feldlinien im wesentlichen parallel zur Strömungsrichtung des Klärschlammes ausgerichtet sind.

Die WO 95/15922 betrifft die Behandlung von Wasser mittels eines durch Permanentmagneten erzeugten statischen Magnetfeldes von wenigstens 6000 Gauss. Das Wasser strömt dabei durch einen Ringspalt zwischen einem positiven und einem negativen Magnetpol, so dass ein homogenes radiales Magnetfeld das durchströmende Wasser beaufschlagt.

Die JP 60-082190 A betrifft die Behandlung von aktiviertem Schlamm mittels Permanentmagneten, die am Umfang eines Behandlungstanks angeordnet sind, um Sauerstoff im Schlamm zu erzeugen. Die Ausrichtung der Feldlinien ist nicht gezeigt.

Die DE 23 61 056 A1 betrifft ein Verfahren zur Vergrößerung der Wachstumsrate von Mikroorganismen in biologischen Abwasseranlagen. Hierzu schlägt die Druckschrift Elektro- oder Permanentmagnete vor. In einer näher beschriebenen Ausführung durchdringen die Feldlinien eines Elektromagneten in Form einer um ein Rohr gewickelten Spule das Abwasser, welches durch das Rohr strömt, homogen in Richtung senkrecht zur Strömungsrichtung.

Aus der FR 2 681 062 A1, der EP 0 616 977 A1, der JP 2003-346274 A und der EP 1 475 353 A1 sind alternierende Ausrichtungen von Permantentmagneten bekannt. Dabei handelt es sich um blockförmige Magnetanordnungen, so dass sich kein Ring von Magneten gleicher Polarität um das Rohr herum erstreckt. Die Anordnungen sollen insbesondere die Kristallisation von Kalzium verhindern oder Wasser sterilisieren.

Das US-Patent 5,161,512 offenbart eine Vorrichtung, in der mehrere Permanentmagnetstreifen gleicher Ausrichtung in axialer Richtung schraubenförmig um ein Rohr gewunden sind.

Überraschenderweise hat sich nun in Versuchen herausgestellt, dass die Beaufschlagung von Biomasse, insbesondere von Klärschlamm, durch ein Permanentmagnetfeld, welches sich von den im Stand der Technik zur Behandlung von Schlamm und Abwasser bekannten radialen oder homogenen Feldern unterscheidet, zu einer deutlichen Erhöhung des aus der Biomasse, insbesondere dem Klärschlamm gewonnenen Heizgas sowie gleichzeitig zu einer deutlichen Reduzierung der verbleibenden Restbiomasse bzw. Restklärschlamms führt.

Es wird angenommen, dass das erfindungsgemäße Magnetfeld durch den von ihm bewirktem deutlich verbesserten Zellaufschluss der beaufschlagten Biomasse eine deutliche Steigerung der Leistung und somit der Wirtschaftlichkeit der Biogasanlage ermöglicht. Im Gegensatz hierzu streben die oben genannten JP 60-082190 A und DE 23 61 056 A1 gerade einer erhöhte Wachstumsrate der Mikroorganismen an. Das bei einer erfindungsgemäßen Vorrichtung bzw. einem erfindungsgemäßen Verfahren verwendet Magnetfeld führt im Gegensatz hierzu zu einer Zerstörung der in der Biomasse, insbesondere dem Klärschlamm vorhandenen Zellen und damit zu dem gewünschten verbesserten Zellaufschluss.

### Aufgabenstellung

Ausgehend von dem genannten Stand der Technik ist es die Aufgabe der vorliegenden Erfindung, dei bei der Ausfaulung von Biomasse, insbesondere von Klärschlamm, anfallende Restbiomasse bzw. den anfallenden Restschlamm zu reduzierten.

### Zusammenfassung der Erfindung

Hierzu ist eine Vorrichtung nach dem Oberbegriff des Anspruchs 1 bzw. ein Verfahren nach dem Oberbegriff des Anspruchs 6 durch deren kennzeichnenden Merkmale weitergebildet.

Erfindungsgemäß umfasst eine Vorrichtung zur Behandlung von Biomasse, insbesondere von Klärschlamm, einen Hohlraum zur Aufnahme der Biomasse bzw. des Klärschlamms, eine erste Magnetanordnung mit einer Mehrzahl von ersten Permanentmagneten und eine zweite Magnetanordnung mit einer Mehrzahl von zweiten Permanentmagneten, wobei alle ersten Permanentmagnete der ersten Magnetanordnung mit ihrem Nordpol zu dem Hohlraum hin und ihrem Südpol von dem Hohlraum weg ausgerichtet am Aussenumfang des Hohlraums angeordnet sind und alle zweiten Permanentmagnete der zweiten Magnetanordnung mit ihrem Südpol zu dem Hohlraum hin und ihrem Nordpol von dem Hohlraum weg ausgerichtet am Aussenumfang des Hohlraums angeordnet sind.

Somit erstrecken sich die Feldlinien von einem Nordpol eines ersten zu einem Südpol wenigstens eines zweiten Permanentmagneten in dem Hohlraum. Durch dieses Magnetfeld werden nicht nur die Zellen in der Biomasse aufgeschlossen, sondern der zelluläre Faulungsprozess wird beschleunigt und intensiviert. Damit erhöht sich vorteilhaft die Menge des nutzbaren Biogases, welches insbesondere als Heizgas verwertet werden kann, in Versuchen um 20-50%. Gleichzeitig reduziert sich auch die anfallende und zu entsorgende Restbiomasse, in Versuchen um 20-30%. Insbesondere reduziert sich unerwünschter Blärschlamm signifikant. Vorteilhaft ergibt sich darüber hinaus eine Verkürzung der Faulzeiten, in Versuchen bis zu 50%.

Gegenüber den einleiten dargestellten Behandlungen mit Ultraschall oder elektromagnetischen Wechselfeldern benötigt eine erfindungsgemässe Vorrichtung keine externe Energie. Sie kann auch - im Gegensatz zur DE 101 18 839 A1 - ohne Betriebsunterbrechung sehr einfach bei bestehenden Abwasserkläranlagen oder anderen Biogasanlagen nachgerüstet werden, wobei die Permanentmagnete ohne bauliche Veränderung der bestehenden Anlage lösbar oder fest an einem Zufluss zum oder dem Faulraum selber angebracht werden können.

Die erste und die zweite Magnetanordnung umfassen je eine Mehrzahl von ersten bzw. zweiten Permanentmagneten, so dass sich im Inneren des Hohlraums das erfindungsgemäße Feld ergibt, welches sich von den im Stand der Technik bekannten Magnetfeldern dahingehend unterscheidet, dass die Feldlinien den Hohlraum nicht vollständig durchdringen, sondern von einem Nordpol eines ersten Permanentmagneten zum Südpol eines zweiten Permanentmagneten reichen. Die ersten und zweiten Permanentmagneten sind in einer axialen Richtung senkrecht zu ihrer Magnetachse alternierend angeordnet, d.h. auf einen ersten Permanentmagnet folgt ein zweiter und umgekehrt. In einer besonders bevorzugten Ausführung sind dabei drei Permanentmagnete der ersten oder zweiten Magnetanordnung und zwei Permanentmagnete der jeweils anderen Magnetanordnung alternierend angeordnet, so dass in der axialen Richtung insgesamt fünf Permanentmagnetreihen vorhanden sind.

In einer Umfangsrichtung senkrecht zu ihrer Magnetachse und der sind axialen Richtung jeweils nur erste oder zweite Permanentmagnete aufeinander-folgend angeordnet. Erfindungsgemäß erstreckt sich in Umfangsrichtung jeweils ein ganzer Ring aus ersten bzw. zweiten Permanentmagneten um den Hohlraum herum.

Es hat sich in Versuchen als vorteilhaft erwiesen, die Permanentmagnete so zu dimensionieren und anzuordnen, dass der magnetische Fluss im Inneren des Hohlraums höchstens 1800 Gauss, insbesondere höchstens 1500 Gaus, und bevorzugt weniger als 800 Gauss beträgt. Durch diese geringen Feldstärken werden Beschädigungen der Anlage, insbesondere Materialermüdung wie bei Vorrichtungen nach dem Stand der Technik, und Beeinträchtigungen des Bedienpersonals durch starke elektromagnetische Strahlung vermieden und gleichzeitig die oben erläuterten Verbesserungen erreicht

Bevorzugt sind die Permanentmagnete stronzium-keramische Magnete. Damit sind extrem lange Lebensdauern ohne signifikantes Nachlassen der magnetischen Wirkung möglich, die Feldstärke nimmt in 90 Jahren um nur ungefähr 1 % ab.

Die erfindungsgemässe Vorrichtung kann gleichermassen an einem Zuführrohr zur Zufuhr von Biomasse, insbesondere von Klärschlamm zu einem Faulraum als auch am Faulraum selber angeordnet sein.

Wird auch das Biogas mittels einer erfindungsgemässen Vorrichtung mit einem Magnetfeld beaufschlagt, so kann der Heizwert des Gases signifikant gesteigert werden, in Versuchen um bis zu 20%.

Als Biomasse kommt insbesondere Klärschlamm, etwa der in einer oder mehreren Reinigungsstufen anfallende Frisch-, Belebt-, Schwimm- und/oder Überschussschlamm in Frage. Gleichermaßen kann die verwendete Biomasse in mehreren Arbeitsstufen verarbeitet werden. Dabei kann aus jeder der Stufen der gesamte Klärschlamm bzw. die gesamte Biomasse oder nur eine Teilmenge davon erfindungsgemäss mit einem Magnetfeld beaufschlagt werden. Der entstehende Restschlamm bzw. die Restbiomasse kann durch ein erfindungsgemässes Verfahren re-vitalisiert und ganz oder teilweise dem zu klärenden Abwasser bzw. anfallenden Klärschlamm wieder zugeführt werden. Ausführungsbeispiel

Weitere Aufgaben, Vorteile und Merkmale der vorliegenden Erfindung ergeben sich aus den Unteransprüchen und den Ausführungsbeispielen. In diesen wird die Erfindung anhand der Behandlung von Klärschlamm näher erläutert. Die Erfindung betrifft jedoch, wie einleitend erwähnt, gleichermaßen auch die Behandlung anderer Biomassen wie etwa in der Landwirtschaft aus Planzenabfällen und/oder Tierfäkalien erzeugte Biomasse. Hierzu zeigt:
Fig. 1 eine Vorrichtung zur Behandlung von Klärschlamm nach einer Ausführung der vorliegenden Erfindung im Längsschnitt;
Fig. 2 einen Querschnitt der Fig. 1 längs der Linie A-B;
Fig. 3 einen Längsschnitt der Fig. 1 längs der Linie C-D mit Feldlinien; und.
Fig. 4A, Fig. 4B einen ersten bzw. zweiten Permanentmagneten.

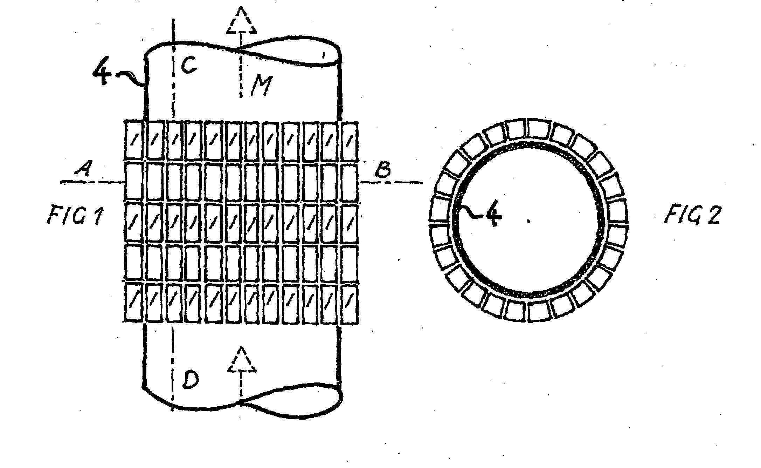

In Fig. 1 durchströmt Klärschlamm M einen als Rohr ausgebildeten Hohlraum 4 in der mit strichliertem Pfeil angedeuteten Richtung. Bei dem Klärschlamm M kann es sich beispielsweise um Frischschlamm, Belebtschlamm, Überschussschlamm oder Schwimmschlamm o.ä. handeln, der, in einer Abwasserkläranlage bei der mechanischen oder biologischen Abwasserreinigung am Rechen, im Vorklärbecken, einem Belebungsbecken oder Nachklärbecken anfällt und einem Faulraum zugeführt wird. Es kann sich jeweils um den gesamten, in einer Stufe anfallenden Klärschlamm handeln, gleichermassen kann jedoch auch nur ein Teil dieses Klärschlammes der erfindungsgemässen Vorrichtung zugeführt werden.

Ein Strömen des Klärschlammes ist nicht notwendig, vielmehr kann er sich auch in Ruhe befinden, beispielsweise in dem Faulraum selber. Eine entsprechende Ausführung der vorliegenden Erfindung ist nicht separate dargestellt, vielmehr bezeichnet in diesem Fall Bezugszeichen 4 in den Fig. 1-Fig. 3 den Faulraums selbst.

Entlang des Umfanges des Hohlraums 4 sind Permanentmagnete 1, 2 wechselweise polarisiert derart angeordnet, dass erste Permanentmagnete 1 mit ihrem Nordpol zum Klärschlamm hin und ihrem Südpol vom Klärschlamm weg ausgerichtet sind, während zweite, mit den ersten alternierend angeordnete Permanentmagnete 2 umgekehrt mit ihrem Südpol zum Klärschlamm hin und ihrem Nordpol vom Klärschlamm weg ausgerichtet sind.

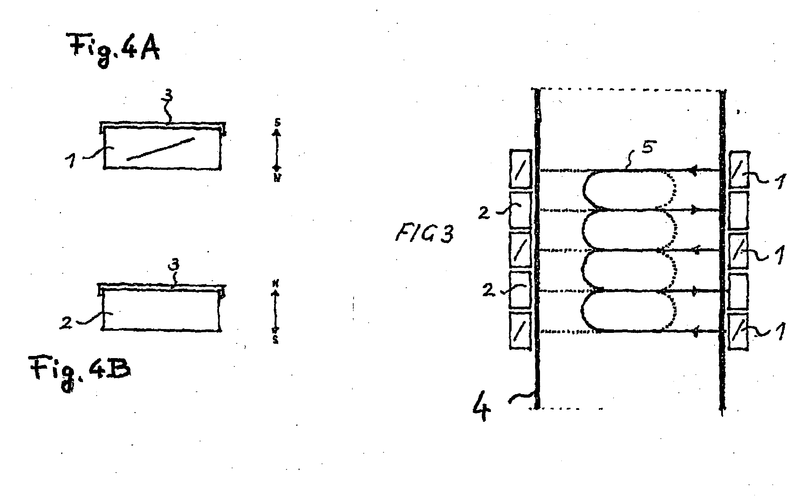

Die alternierende Anordnung erfolgt, wie in Fig. 1, Fig. 3 gezeigt, in axialer Richtung des Hohlraumes 4, so dass sich in axialer Richtung ein erster und ein zweiter Permanentmagnet abwechseln.

Durch die alternierende Anordnung von wechselseitig polarisierten Permanentmagneten 1, 2 ergeben sich die in Fig. 3 schematisch angedeuteten Feldlinien 5, die im Inneren des Hohlraumes 4 jeweils von dem dem Klärschlamm zugewandten Nordpol eines ersten Permanentmagneten 1 zu dem dem Klärschlamm zugewandten Südpol des benachbarten zweiten Permanentmagneten 2 bzw. zu den dem Klärschlamm zugewandten Südpolen der benachbarten zweiten Permanentmagnete 2 verlaufen. Sind die ersten und zweiten Magneten, wie in Fig. 2 dargestellt, jeweils entlang des gesamten Umfangs des Hohlraumes 4 verteilt angeordnet, so durchdringt das sich ergebende Magnetfeld den Klärschlamm gleichmässig und besonders vorteilhaft.

Bevorzugt sind die ersten und zweiten Permanentmagnete so dimensioniert und angeordnet, dass sich eine magnetischer Fluss von etwa 1500 Gauss (0,15T) ergibt, besonders bevorzugt ein magnetischer Fluss von etwa 1800 Gauss (0,18T). Die einzelnen Permanentmagnete können an ihrem dem Klärschlamm abgewandten äusseren Ende mit einer Abdeckung 3 aus Blech, Kunststoff oder dergleichen versehen sein, um sie vor den rauhen Umgebungsbedingungen bei Kläranlagen zu schützen, Sind die Abdeckungen magnetisierbar bzw. magnetisch, so verstärken sie vorteilhaft den in Fig. 3 dargestellten magnetischen Fluss.

Die ersten und zweiten Permanentmagneten 1, 2 können fest oder lösbar mit dem Hohlraum 4 verbunden sein, beispielsweise durch Kleben, Schrauben, Anpressen mittels Spanngurten oder dergleichen. Bevorzugt weisen die dem Klärschlamm zugewandten Pole eine der Aussenwand des Hohlraumes 4 entsprechende Form auf. Die Magnete können auch einzeln oder in Gruppen in Kunststoff oder dergleichen eingegossen sein (nicht dargestellt).

Setzt man den Klärschlamm, wie in Fig. 1 gezeigt, vor der Zuführung in den Faulraum oder im Faulraum selber, mittels der erfindungsgemässen Anordnung der Permanentmagnete einem sich daraus ergebenden Magnetfeld aus, so führt diens, selbst bei den bevorzugten geringen Feldstärken, vorteilhaft zu einer Zerstörung der Zellwände der Mikroorganismen und einem beschleunigten zellulären Zersetzungsprozess. Dies steigert die biologische Aktivität erheblich - in Versuchen ergab sich mit einer Vorrichtung nach der hier dargestellten Ausführung der vorliegenden Erfindung eine Reduzierung des Restschlammmes von 20-30%. Die Menge des aus dem Klärschlamm gewonnenen Heizgas konnte um 20-50% gesteigert werden. Schließlich konnte auch die Faulzeit des Klärschlamms im Faulraum um bis zu 50% reduziert werden.

Auch das aus dem Klärschlamm gewonnene Biogas kann vorteilhaft in ähnlicher Weise einem Magnetfeld ausgesetzt werden. Die Anordnung entspricht den Fig. 1-Fig. 3, wobei der Hohlraum 4 nun nicht mehr von Klärschlamm, sondern von Heizgas M durchströmt wird Durch die oben beschriebene Anordnung von ersten und zweiten Permanentmagneten 1, 2 wird ein gleichmässiges Magnetfeld erzeugt, durch dessen Einwirken sich der Heizwert des Heizgases M signifikant erhöht, in versuchen bis 20%.

Wie bei Kläranlagen bekannt, kann der Restschlamm dem Prozess auch wieder ganz oder teilweise zugeführt werden. Die Beaufschlagung mit einem Magnetfeld durch die efindungsgemässe Vorrichtung re-vitalisiert diesen Restschlamm, so dass sich die Leistungsfähigkeit der Kläranlage verbessert. In einer nicht separat dargestellten Ausführung der vorliegenden Erfindung wird bei der Rückführung von Restschlamm in die Kläranlage eine erfindungsgemässe Vorrichtung, wie sie in Zusammenhang mit Fig. 1-Fig. 3 beschrieben wurde, von Restschlamm M durchströmt, der dadurch re-vitalisiert wird.

## Patentansprüche

1. Vorrichtung zur Behandlung von Biomasse, insbesondere von Klärschlamm, die umfasst:
einen Hohlraum (4) zur Aufnahme der Biomasse (M);
eine erste Magnetanordnung mit einer Mehrzahl von ersten Permanentmagneten
(1); und
eine zweite Magnetanordnung mit einer Mehrzahl von zweiten Permanentmagneten (2);
wobei alle ersten Permanentmagnet (1) der ersten Magnetanordnung mit ihrem Nordpol zu dem Hohlraum (4) hin und ihrem Südpol von dem Hohlraum (4) weg ausgerichtet am Außenumfang des Hohlraums angeordnet sind; und alle zweiten Permanentmagnet (2) der zweiten Magnetanordnung mit ihrem Südpol zu dem Hohlraum (4) hin und ihrem Nordpol von dem Hohlraum (4) weg ausgerichtet am Außenumfang des Hohlraums angeordnet sind, so dass sich die Feldlinien von einem Nordpol eines ersten zu einem Südpol wenigstens eines zweiten Permanentmagneten in dem Hohlraum (4) erstrecken,
wobei die ersten und zweiten Permanentmagneten (1, 2) in einer axialen Richtung senkrecht zu ihrer Magnetachse alternierend angeordnet sind,
wobei in einer Umfangsrichtung senkrecht zu der Magnetachse der Permanentmagnete und der axialen Richtung jeweils nur erste oder zweite Permanentmagnete aufeinanderfolgend angeordnet sind,
**dadurch gekennzeichnet, dass**
sich in Umfangsrichtung jeweils ein ganzer Ring aus ersten bzw. zweiten Permanentmagneten um den Hohlraum herum erstreckt.

2. Vorrichtung nach Anspruch 1, **dadurch gekennzeichnet, dass** in der axialen Richtung drei Permanentmagnete der ersten oder zweiten Magnetanordnung und zwei Permanentmagnete der jeweils anderen Magnetanordnung alternierend angeordnet sind.

3. Vorrichtung nach einem der vorhergehenden Ansprüche, **dadurch gekennzeichnet, dass** die ersten und/oder zweiten Permanentmagneten stronzium-keramische Magnete sind.

4. Vorrichtung nach einem der vorhergehenden Ansprüche, **dadurch gekennzeichnet, dass** der Hohlraum ein Zuführrohr zur Zufuhr von Biomasse zu einem Faulraum ist.

5. Vorrichtung nach einem der Ansprüche 1-3, **dadurch gekennzeichnet, dass** der Hohlraum ein Faulraum ist.

6. Verfahren zur Behandlung von Biomasse, insbesondere von Klärschlamm, das umfasst:
Zufuhr von Biomasse in einen Faulraum; Ausfaulenlassen der Biomasse im Faulraum; Abfuhr der dabei entstehenden Restbiomasse und des dabei entstehenden Biogases; **dadurch gekennzeichnet, dass** bei der Zufuhr und/oder im Faulraum die Biomasse mittels einer Vorrichtung nach einem der vorhergehenden Ansprüche mit einem Magnetfeld beaufschlagt wird.

7. Verfahren nach Anspruch 6, **dadurch gekennzeichnet, dass** auch das Biogas mittels einer Vorrichtung mit einem Magnetfeld beaufschlagt wird, die einer Vorrichtung nach einem der Ansprüche 1-5 entspricht, wobei anstelle der Biomasse das Biogas im Hohlraum aufgenommen wird.

8. Verfahren nach Anspruch 6 oder 7, **dadurch gekennzeichnet, dass** als Biomasse Klärschlamm, insbesondere der in einer oder mehreren Reinigungsstufen anfallende Frisch-, Belebt-, Schwimm- und/oder Überschussschlamm und/oder das bei der Vorreinigung anfallende Rechengut und/oder der bei der Ausfaulung entstandene Restschlamm ganz oder teilweise zugeführt wird.

9. Verfahren nach einem der vorhergehenden Ansprüche 6 bis 8, **dadurch gekennzeichnet, dass** der magnetische Fluss im Inneren des Hohlraums höchstens 1800 Gauss, insbesondere höchstens 1500 Gaus, und bevorzugt weniger als 800 Gauss beträgt.

## Claims

1. A device for the treatment of biomass, in particular sewage sludge, comprising a cavity (4) for accommodating the biomass (M);
a first magnet arrangment having a plurality of first permanent magnets (1); and
a second magnet arrangement having a plurality of second permanent magnets (2);
all first permanent magnets (1) of the first magnet arrangement being situated on the outer periphery of the cavity, with their north poles oriented toward the cavity (4) and their south poles oriented away from the cavity (4); and
all second permanent magnets (2) of the second magnet arrangement being situated on the outer periphery of the cavity with their south poles oriented toward the cavity (4) and their north poles oriented away from the cavity (4),
so that the flux lines from a north pole of a first permanent magnet to a south pole of at least one second permanent magnet run in the cavity (4),
wherein the first and second permanent magnets (1, 2) are situated in alternating fashion in the axial direction perpendicular to their magnetic axis;
wherein in the peripheral direction, perpendicular to the magnetic axis of the permanent magnets and to the axial direction, only first or second permanent magnets respectively are arranged one after the other **characterized in that** in the peripheral direction a complete ring of first or, respectively, second permanent magnets extends around the cavity.

2. The device as recited in Claim 1, **characterized in that** in the axial direction three permanent magnets of the first or second magnet arrangement and two permanent magnets of the respective other magnet arrangement are situated in alternating fashion.

3. The device as recited in one of the preceding claims, **characterized in that** the first and/or second permanent magnets are strontium-ceramic magnets.

4. The device as recited in one of the preceding claims, **characterized in that** the cavity is a supply pipe for supplying biomass to a digesting chamber.

5. The device as recited in one of Claims 1-3, **characterized in that** the cavity is a digesting chamber.

6. A method for treating biomass, in particular sewage sludge, comprising:
feeding of biomass into a digesting chamber;
digesting of the biomass in the digesting chamber;
drainage of the resulting residual biomass and of the resulting biogas;
**characterized in that**
during the feeding into the digesting chamber and/or within the digesting chamber, the biomass is charged with a magnetic field using a device according to one of the preceding claims.

7. The method as recited in Claim 6, **characterized in that** the biogas is also charged with a magnetic field using a device corresponding to a device as recited in one of Claims 1-5, the biogas being held in the cavity instead of the biomass.

8. The method as recited in Claim 6 or 7, **characterized in that** as biomass sewage sludge is supplied wholly or in part, in particular the fresh sludge, activated sludge, scum, and/or excess sludge that arises in one or more cleaning stages, and/or the screened material that arises during pre-cleaning, and/or the residual sludge that arises during digesting.

9. The method as recited in one of the preceding claims 6 to 8, **characterized in that** the magnetic flux inside the cavity is at most 1800 gauss, in particular at most 1500 gauss, preferably less than 800 gauss.

## Revendications

1. Dispositif pour le traitement de biomasse, notamment de boues de curage, qui comprend :
une chambre (4) destinée à recevoir la biomasse (M) ;
un premier agencement d'aimant avec une pluralité de premiers aimants permanents (1) ; et
un deuxième agencement d'aimant avec une pluralité de deuxièmes aimants permanents (2) ;
dispositif
dans lequel tous Les premiers aimants permanents (1) du premier agencement d'aimant sont agencés à la périphérie extérieure de la chambre, en étant orientés avec leur pôle nord dirigé vers la chambre (4) et leur pôle sud s'éloignant de la chambre (4), et tous les deuxièmes aimants permanents (2) du deuxième agencement d'aimant sont agencés à la périphérie extérieure de la chambre, en étant orientés avec leur pôle sud dirigé vers la chambre (4) et leur pôle nord s'éloignant de la chambre (4), de sorte que les lignes de champ d'un pôle nord d'un premier vers un pôle sud d'au moins un deuxième aimant permanent (1, 2), s'étendent dans la chambre (4) ,
dans lequel les premiers et les deuxièmes aimants permanents (1, 2) sont agencés de manière alternée dans une direction axiale perpendiculaire à leur axe magnétique,
et dans lequel, dans une direction périphérique perpendiculaire à l'axe magnétique des aimants permanents et à la direction axiale, ne sont respectivement agencés successivement que des premiers ou des deuxièmes aimants permanents,
**caractérisé en ce que** dans la direction périphérique autour de la chambre, s'étend un anneau à chaque fois complet de premiers respectivement de deuxièmes aimants permanents.

2. Dispositif selon la revendication 1, **caractérisé en ce que** dans la direction axiale sont agencés de manière alternée trois aimants permanents du premier ou du deuxième agencement d'aimant et deux aimants permanents respectivement de l'autre agencement d'aimant.

3. Dispositif selon l'une des revendications précédentes, **caractérisé en ce que** les premiers et/ou les deuxièmes aimants permanents sont des aimants céramiques-strontium.

4. Dispositif selon l'une des revendications précédentes, **caractérisé en ce que** la chambre est un tube d'amenée pour l'amenée de biomasse à une chambre de putréfaction ou de digestion.

5. Dispositif selon l'une des revendications 1 - 3, **caractérisé en ce que** la chambre est une chambre de putréfaction ou de digestion.

6. Procédé de traitement de biomasse, notamment de boues de curage, qui comprend :
l'amenée de la biomasse à une chambre de putréfaction ou de digestion ; une phase de putréfaction de la biomasse dans la chambre de putréfaction ou de digestion ; l'évacuation de la biomasse résiduelle produite à cette occasion et du biogaz produit à cette occasion,
**caractérisé en ce que** lors de l'amenée et/ou dans la chambre de putréfaction ou de digestion, la biomasse est soumise à l'action d'un champ magnétique au moyen d'un dispositif selon l'une des revendications précédentes.

7. Procédé selon la revendication 6, **caractérisé en ce que** le biogaz est également soumis à l'action d'un champ magnétique au moyen d'un dispositif qui correspond à un dispositif selon l'une des revendications 1 - 5, le biogaz étant reçu dans la chambre à la place de la biomasse.

8. Procédé selon la revendication 6 ou 7, **caractérisé en ce que** la biomasse amenée en totalité ou en partie est constituée par des boues de curage, notamment des boues fraîches, activées, flottantes et/ou excédentaires produites au cours d'une ou de plusieurs étapes d'épuration, et/ou par les matières retenues par la grille lors d'une épuration préliminaire, et/ou par les boues résiduelles produites lors de la putréfaction ou de la digestion.

9. Procédé selon l'une des revendications précédentes 6 à 8, **caractérisé en ce que** le flux magnétique à l'intérieur de la chambre à une valeur d'au plus 1800 Gauss, notamment au plus de 1500 Gauss, et de préférence de moins de 800 Gauss.
